(19) Europäisches Patentamt
European Patent Office
Office européen des brevets

(11) EP 1 695 694 A2

(12) # DEMANDE DE BREVET EUROPEEN

(43) Date de publication:
**30.08.2006 Bulletin 2006/35**

(51) Int Cl.:
***A61K 8/92*** *(2006.01)*

(21) Numéro de dépôt: **06100747.2**

(22) Date de dépôt: **24.01.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **04.02.2005 FR 0550335**

(71) Demandeur: **l'Oreal SA**
**75008 Paris (FR)**

(72) Inventeurs:
- **Jager Lezer, Nathalie**
**Verrieres-le-Buisson 91370 (FR)**
- **Auguste, M. frédéric**
**Chevilly Larue 94550 (FR)**

(74) Mandataire: **Duvert, Sandra**
**L'ORÉAL - DIPI**
**25-29 Quai Aulagnier**
**95600 Asnieres (FR)**

(54) **Composition de revêtement des fibres kératiniques comprenant une cire aprotique**

(57) L'invention a pour objet une composition de revêtement des fibres kératiniques comprenant un milieu aqueux continu cosmétiquement acceptable caractérisée en ce qu'elle comprend au moins une cire aprotique en une teneur supérieure à 25% en poids par rapport au poids total de la composition, ladite composition ayant un extrait sec supérieur ou égal à 40%.
L'invention a également pour objet une composition de revêtement des fibres kératiniques comprenant un milieu aqueux continu cosmétiquement acceptable caractérisée en ce qu'elle comprend au moins 20% d'au moins une cire aprotique et au moins un polymère filmogène choisi parmi les dérivés cellulosiques

L'invention a également pour objet l'utilisation d'une telle composition pour obtenir un maquillage chargeant des fibres kératiniques et/ou un dépôt lisse et homogène sur lesdites fibres kératiniques.

EP 1 695 694 A2

## Description

La présente invention a pour objet une composition cosmétique de revêtement des fibres kératiniques.

La composition selon l'invention peut être une composition de maquillage, encore appelée mascara, une base de maquillage des fibres kératiniques ou base-coat, une composition à appliquer sur un maquillage, dite encore top-coat, ou bien encore une composition de soin des fibres kératiniques.
De préférence, la composition selon l'invention est une composition non rincée.

Plus spécialement, la composition selon l'invention est un mascara.
Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de soin cosmétique des cils. Le mascara est plus particulièrement destiné aux cils d'êtres humains, mais également aux faux-cils.

Les compositions de maquillage pour les yeux, encore appelées « mascara » pour les cils sont généralement constituées d'une cire ou mélange de cires dispersée(s) à l'aide d'au moins un tensioactif dans une phase aqueuse contenant, par ailleurs, des polymères et des pigments.
C'est généralement à travers le choix qualitatif et quantitatif des cires et polymères que sont ajustées les spécificités d'application recherchées pour les compositions de maquillage, comme par exemple leur fluidité, leur pouvoir couvrant et/ou leur pouvoir recourbant. Ainsi, il est possible de réaliser diverses compositions qui, appliquées notamment sur les cils, induisent des effets variés de type allongement, recourbement et/ou épaississement (effet chargeant).

La présente invention vise plus particulièrement à proposer une composition utile pour réaliser un maquillage épais des fibres kératiniques et notamment des cils, dit encore maquillage chargeant. Au sens de la présente invention, le terme fibres kératiniques couvre les cheveux, les cils et les sourcils et s'étend également aux postiches et faux-cils synthétiques.

Il est connu de l'art antérieur que plus la teneur en solides (apportée en partie par une phase grasse constituée par exemple, d'une ou plusieurs cires ou d'un ou plusieurs polymères lipophiles) dans une composition va augmenter, plus le dépôt de matière sur le cil va être important et donc plus le résultat obtenu sera volumateur.

Néanmoins, l'augmentation de la teneur en solides dans une composition, telle qu'une émulsion ou dispersion entraîne une augmentation de la consistance du produit obtenu et donc une application sur les cils délicate et difficile car le produit est épais, visqueux, il se dépose difficilement, de façon hétérogène et par paquets et le maquillage ainsi obtenu présente un aspect granuleux, non lisse ; le maquillage n'est pas homogène et a un aspect inesthétique.

Un autre moyen d'augmenter la teneur en solides est d'incorporer des particules solides comme des charges ou des pigments mais l'augmentation de la consistance limite également le pourcentage maximum en solides, de plus l'utilisation de particules solides en quantité importante ne favorise pas le dépôt homogène et lisse en raison non seulement de la consistance mais aussi de la taille des particules introduites qui donne un aspect granuleux et non lisse au dépôt.
C'est généralement le cas des mascaras dits volumateurs qui sont difficiles à appliquer et donnent un maquillage hétérogène.

Il est donc difficile d'obtenir une composition de maquillage des fibres kératiniques, comprenant une forte teneur en solides et donc un effet volumateur satisfaisant, présentant une application facile et un dépôt homogène.

De manière inattendue, il a ainsi été constaté qu'il était possible de préparer des compositions présentant un extrait sec élevé et permettant un maquillage épaississant des fibres kératiniques et un dépôt lisse et homogène sur lesdites fibres grâce à la mise en oeuvre, dans ces compositions, d'une cire aprotique en une certaine teneur.
Au sens de la présente invention, on entend qualifier par le terme « chargeant » la notion d'un maquillage épais et volumateur des fibres kératiniques, en particulier des cils.

En particulier, les compositions selon l'invention présentent avantageusement un extrait sec supérieur ou égal à 40 % en poids, de préférence supérieur ou égal à 42% en poids, mieux, supérieur ou égal à 45% et encore mieux supérieur ou égal à 47% par rapport au poids total de la composition, pouvant aller jusqu'à 70% en poids.

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec » des compositions selon l'invention, est mesurée par échauffement de l'échantillon par des rayons infrarouges de 2 $\mu$m à 3,5 $\mu$m de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.
Le protocole de mesure est le suivant :

On étale environ 1 g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.
La teneur en matière sèche est calculée de la manière suivante :

Extrait Sec = 100 x (masse sèche / masse humide).

L'invention a ainsi pour objet, selon l'un de ses aspects, une composition de revêtement des fibres kératiniques comprenant un milieu aqueux continu cosmétiquement acceptable et au moins une cire aprotique en une teneur supérieure à 25% en poids par rapport au poids total de la composition, ladite composition ayant un extrait sec supérieur ou égal à 40%.

Selon un autre aspect, l'invention a également pour objet une composition de revêtement des fibres kératiniques comprenant un milieu aqueux continu cosmétiquement acceptable caractérisée en ce qu'elle comprend au moins 20% d'au moins une cire aprotique et au moins un polymère filmogène choisi parmi les dérivés cellulosiques.

Par composition à milieu aqueux « continu », on entend que la composition présente une conductivité, mesurée à 25°C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La présente invention vise également un procédé de maquillage des fibres kératiniques,
caractérisé par le fait que l'on applique sur lesdites fibres une composition conforme à l'invention.

Elle se rapporte en outre à l'utilisation d'une composition conforme à l'invention pour obtenir un maquillage chargeant des fibres kératiniques et notamment des cils et des sourcils et/ou un dépôt lisse et homogènes sur lesdites fibres.

La présente invention a aussi pour objet l'utilisation, dans une composition comprenant un milieu aqueux continu cosmétiquement acceptable, d'au moins une cire aprotique pour obtenir un maquillage chargeant des fibres kératiniques et notamment des cils et des sourcils et un dépôt lisse et homogène sur lesdites fibres.

**Cire aprotique**

Dans le cadre de la présente invention, une cire peut être définie comme étant un composé lipophile, solide à température ambiante (25 °C), à changement d'état solide/liquide réversible, ayant un point de fusion supérieur ou égal à 30 °C pouvant aller jusqu'à 120 °C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur à 45° environ, mieux supérieur ou égal à 50°C et en particulier supérieur ou égal à 55 °C.
Le point de fusion de la cire peut être mesuré à l'aide d'un calorimètre à balayage différentiel (D.S.C.), par exemple le calorimètre vendu sous la dénomination MDSC 2929 par la société TA Instruments.
Le protocole de mesure est le suivant :

Un échantillon de 5 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

On entend par "cire aprotique", une cire comprenant peu ou ne comprenant pas d'atome d'hydrogène lié à un atome fortement électronégatif tel que O ou N.
De préférence, les cires aprotiques sont choisies parmi les cires apolaires, c'est-à-dire des cires constituées uniquement de molécules ne comportant que des atomes de carbone et d'hydrogène dans leur structure chimique, autrement dit

ne comprenant pas d'hétéroatomes (tels que O, N, P) .

On peut citer à titre d'exemples de cires aprotiques, en particulier apolaires, les cires de paraffine, les cires microcristallines, l'ozokérite, la cérésine et les cires synthétiques comme les cires de polyméthylène, de polyéthylène, de propylène et leurs copolymères éthylène/propylène ou encore les cires de Fischer-Tropsch et leurs mélanges.

Peuvent être considérées comme aprotiques au sens de la présente invention, les cires obtenues par esterification ou modifiées par esterification et qui peuvent comprendre des groupes OH résiduels en fonction du rendement de l'esterification. De telles cires sont par exemple la cire issue de la réaction d'un acide gras sur un polyol ramifié de type ditrimethylol comme par exemple celles commercialisées sous la dénomination HEST par la société Heterene. On peut également citer les cires modifiées siliconées comme la cire de candellila siliconée commercialisée par Koster Keunen sous la dénomination Siliconyl candellila.

Sont également considérées comme aprotiques les cires obtenues par hydrogénation catalytique d'huiles animales ou végétales ayant des chaînes grasses, linéaires ou ramifiées, en C8-C32 telles que l'huile de jojoba hydrogénée, l'huile de tournesol hydrogénée, l'huile de coprah hydrogénée ou encore la cire obtenue par hydrogénation d'huile d'olive estérifiée avec l'alcool stéarylique.

Par opposition, sont considérées comme protiques les cires hydrocarbonées comme la cire d'abeille, la cire de lanoline ; la cire d'orange, la cire de citron, la cire de son de riz, la cire de Carnauba, la cire de Candellila, la cire d'Ouricury, la cire du Japon, la cire de Berry, la cire de shellac et la cire de sumac ; la cire de Montan, l'huile de ricin hydrogénée, l'huile de lanoline hydrogénée, les cires issues de la réaction d'acides gras sur des carbohydrates, comme les disaccharides de type sucrose, telles que le polybéhénate de sucrose, commercialisé par Croda sous la dénomination Cromaderm B, les cires hydroxyesters comme par exemple la cire (hydroxystéaryloxy)stéarate d'alkyle en $C_{20}$-$C_{40}$ telles que celles vendues sous les dénominations « Kester Wax K 82 P® » et « Kester Wax K 80 P® » par la société KOSTER KEUNEN

Les cires aprotiques synthétiques conviennent tout particulièrement telles que les cires de paraffine, les cires de cérésine et d'ozokérite, les cires de polyméthylène et en particulier les cires Cirebelle 303® et Cirebelle 505® fabriquée par la société SASOL, de polyéthylène, de propylène et leurs copolymères éthylène/propylène, les cires de Fischer-Tropsch et leurs mélanges.

On peut également citer les cires de polyoléfine telles que celles issues de la polymérisation, et notamment d'homopolymérisation, d'alpha-oléfine répondant à la formule générale : $R-CH=CH_2$ dans laquelle R désigne un radical alkyle, de préférence alkyl linéaire, ayant de 10 à 50 atomes de carbone, et de préférence de 25 à 50 atomes de carbone.

On entend par homopolymérisation d'alpha-oléfine la polymérisation de monomères consistant essentiellement en une alpha-oléfine ou un mélange d'alpha-oléfine.

De telles cires de polyoléfines peuvent avoir un poids moléculaire moyen en nombre allant de 400 à 3000, de préférence de 2000 à 3000 et mieux de 2500 à 2700.

De telles cires de polyoléfine sont décrites dans les brevets US-A-4060569 et US-A-4239546. Ces cires sont notamment vendues sous la dénomination de "PERFORMA V® 103", "PERFORMA V® 253", "PERFORMA V® 260" par la société PETROLITE.

Avantageusement, la ou les cire(s) aprotique(s) peu(ven)t être présente(s) en une teneur allant de 20 à 60% en poids, mieux de 25 à 60 % en poids, notamment de 27 à 50 % en poids, et en particulier de 28 à 45 % en poids par rapport au poids total de la composition.

Selon un autre aspect, la composition selon l'invention comprend moins de 5 % en poids de cire(s) protique(s), telles que définies plus haut, par rapport au poids total de la composition, de préférence moins de 4%, mieux moins de 3% et encore mieux moins de 2% en poids.

C'est pourquoi l'invention a encore pour objet une composition de revêtement des fibres kératiniques comprenant un milieu aqueux continu cosmétiquement acceptable caractérisée en ce qu'elle comprend moins de 5 % en poids de cire protique, par rapport au poids total de la composition.

De préférence, la composition selon l'invention est exempte de cire protique.

## Milieu aqueux

Le milieu aqueux continu cosmétiquement acceptable de la composition selon l'invention peut être constituée essentiellement d'eau ; il peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.

Le milieu aqueux (eau et éventuellement le solvant miscible à l'eau) peut être présent en une teneur allant de 0,1 % à 95 % en poids, par rapport au poids total de la composition, de préférence allant de 1 % à 80 % en poids

Système émulsionnant

La composition selon l'invention peut contenir des agents tensioactifs émulsionnants présents notamment en une proportion allant de 0,1 à 30 % en poids par rapport au poids total de la composition, mieux de 1 à 15 % et mieux de 2 à 10 %.
Selon l'invention, on utilise généralement un émulsionnant choisi de manière appropriée pour l'obtention d'une émulsion huile-dans-eau. En particulier, on peut utiliser un émulsionnant possédant à 25 °C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Ces agents tensioactifs peuvent être choisis parmi des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.
Les tensioactifs utilisés préférentiellement dans la composition selon l'invention sont choisis parmi :

a) les agents tensioactifs non ioniques de HLB supérieur ou égal à 8 à 25 °C, utilisés seuls ou en mélange; on peut citer notamment :

les éthers oxyéthylénés et/ou oxypropylénés (pouvant comporter de 1 à 150 groupes oxyéthylénés et/ou oxypropylénés) de glycérol ;
les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7® par SHELL CHEMICALS,
les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,
les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60® par la société UNIQUEMA,
la diméthicone copolyol, telle que celle vendue sous la dénomination Q2-5220® par la société DOW CORNING,
la diméthicone copolyol benzoate (FINSOLV SLB 101® et 201® de la société FINTEX),
les copolymères d'oxyde propylène et d'oxyde d'éthylène, également appelés polycondensats OE/OP,
et leurs mélanges.
Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH2\text{-}CH2)_a\text{-}(O\text{-}CH(CH3)\text{-}CH2)_b\text{-}(O\text{-}CH2\text{-}CH2)_a\text{-}OH,$$

formule dans laquelle a va de 2 à 120, et b va de 1 à 100.
Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus

sous les dénominations SYNPERONIC® comme les SYNPERONIC PE/ L44® et SYNPERONIC PE/F127® par la société ICI.

b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :

les esters et éthers d'oses tels que les stéarate de sucrose, cocoate de sucrose, stéarate de sorbitan et leurs mélanges comme l'Arlatone 2121® commercialisé par la société ICI;
les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
le mélange de cyclométhicone/diméthicone copolyol vendu sous la dénomination Q2-3225C® par la société DOW CORNING.

c) Les tensioactifs anioniques tels que :

les sels d'acides gras en C16-C30 notamment ceux dérivant des amines, comme le stéarate de triéthanolamine et/ou le stéarate d'amino-2 méthyl-2 propane di-ol-1,3;

les sels d'acides gras polyoxyéthylénés notamment ceux dérivant des amines ou les sels alcalins, et leurs mélanges ;
les esters phosphoriques et leurs sels tels que le "DEA oleth-10 phosphate" (Crodafos N 10N de la société CRODA) ou le phosphate de monocétyle monopotassique (Amphisol K de Givaudan) ;
les sulfosuccinates tels que le "Disodium PEG-5 citrate lauryl sulfosuccinate" et le "Disodium ricinoleamido MEA sulfosuccinate" ;
les alkyléthersulfates tels que le lauryl éther sulfate de sodium ;
les iséthionates ;
les acylglutamates tels que le "Disodium hydrogenated tallow glutamate" (AMISOFT HS-21 R® commercialisé par la société AJINOMOTO) et leurs mélanges.

Convient tout particulièrement à l'invention, le stéarate de triéthanolamine et/ou d'amino-2 méthyl-2 propane di-ol-1,3.. Ce dernier est généralement obtenu par simple mélange de l'acide stéarique et de la triéthanolamine et/ou de l'amino-2 méthyl-2 propane di-ol-1,3.

Les compositions selon l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

**Polymère filmogène**

La composition selon l'invention peut comprendre un polymère filmogène. Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$- Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliqueset les monomère styréniques . En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle. Comme monomère styrèniques, on peut citer le styrène, et l'alpha méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.
La composition peut comprendre un polymère filmogène hydrosoluble choisi parmi :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique;
- les polymères d'origine naturelle, éventuellement modifiés, tels que :
  - les gommes arabiques, la gomme de guar, les dérivés du xanthane, la gomme de karaya ;
  - les alginates et les carraghénanes ;
  - les glycoaminoglycanes, l'acide hyaluronique et ses dérivés ;
  - la résine shellac, la gomme de sandaraque, les dammars, les élémis, les copals ;
  - l'acide désoxyribonucléïque ;
  - les muccopolysaccharides tels que l'acide hyaluronique, les chondroïtines sulfate, et leurs mélanges.

Selon un mode de réalisation avantageux, la composition comprend au moins un polymère filmogène choisi parmi les polymères cellulosiques tel que les alkylcelluloses tels que la méthylcellulose, l'éthylcellulose les hydroxyalkylcelluloses tel que l'hydroxyéthylcellulose, l'hydroxypropylcellulose l'éthylhydroxyéthylcellulose, les carboxyalkylcelluloses tels que la carboxyméthylcellulose,
**[0052]** Selon un mode de réalisation particulier de l'invention, la composition comprend au moins un polymère filmogène choisi parmi les dérivés cellulosiques et une cire aprotique présente en une teneur d'au moins 20% en poids par rapport au poids total de la composition.
C'est pourquoi l'invention a encore pour objet une composition de revêtement des fibres kératiniques comprenant un milieu aqueux continu cosmétiquement acceptable caractérisée en ce qu'elle comprend au moins une cire aprotique en une teneur supérieure ou égale à 20% en poids par rapport au pois total de la composition et au moins un polymère filmogène choisi parmi les polymères cellulosiques.
Le polymère filmogène peut être également présent dans la composition sous la forme de particules en dispersion dans une phase aqueuse ou dans une phase solvant non aqueuse (milieu organique liquide de la composition), connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations Neocryl XK-90® , Neocryl A-1070® , Neocryl A-1090® , Neocryl BT-62® , Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer ou encore les dispersions aqueuses de polyuréthane vendues sous les dénominations Neorez R-981® et Neorez R-974® par la société AVECIA-NEORESINS, les Avalure UR-405® , Avalure UR-410® , Avalure UR-425® , Avalure UR-450® , Sancure 875® , Sancure 861® , Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.
La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.
Les compositions revendiquées peuvent également contenir des ingrédients couramment utilisés dans le domaine du maquillage des fibres kératiniques.
La composition selon l'invention peut notamment comprendre une ou plusieurs huiles, de préférence aprotiques. L'huile peut être présente dans la composition selon l'invention en une teneur allant de 0,1 % à 60 % en poids, de préférence de 0,1 % à 30 % en poids par rapport au poids total de la composition.
L'huile peut être choisie parmi les huiles volatiles et/ou les huile non volatiles, et leurs mélanges. La composition comprend avantageusement au moins une huile volatile.
Par " huile volatile", on entend au sens de l'invention une huile susceptible de s'évaporer au contact de la peau

ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).
Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).
Ces huiles peuvent être des huiles hydrocarbonées, des huiles siliconées, des huiles fluorées, ou leurs mélanges.
On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.
Comme huiles volatiles, on peut aussi utiliser les silicones volatiles, comme par exemple les huiles de silicones linéaires ou cycliques volatiles, notamment celles ayant une viscosité $\leq$ 8 centistokes ($8\ 10^{-6}\ m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
On peut également citer les huiles linéaires alkyltrisiloxanes volatiles de formule générale (1)

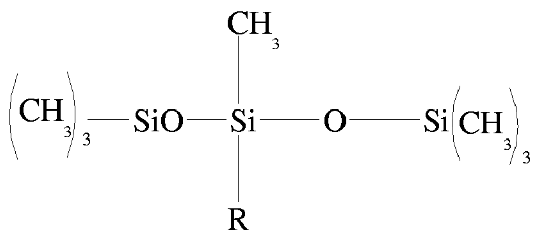

où R représente un groupe alkyle comprenant de 2 à 4 atomes de carbone et dont un ou plusieurs atomes d'hydrogène peuvent être substitués par un atome de fluor ou de chlore.
Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.
On peut également utiliser des solvants volatils fluorés tels que le nonafluorométhoxybutane ou le perfluorométhylcyclopentane.
La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin,

de sésame, de maïs, d'abricot, de karité, d'avocat, d'olive, de soja, l'huile d'amande douce, de palme, de coton, de noisette, de macadamia, de jojoba, de luzerne, de pavot, de potimarron, de sésame, de courge, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1 COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit $\geq$ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates ; les esters hydroxylés comme le lactate d'isostéaryle, le malate de di-isostéaryle ; et les esters du pentaérythritol ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;
Les huiles fluorées utilisables dans l'invention sont notamment des huiles fluorosiliconées, des polyéthers fluorés, des silicones fluorées telles que décrit dans le document EP-A-847752.

Additifs

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le $\beta$-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.
La composition selon l'invention peut également comprendre une charge choisie parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® (Orgasol de chez Atochem), de poly-$\beta$-alanine et de polyéthylène, les poudres de polymères de tétrafluoroéthylène comme le Téflon® , la lauroyl-lysine, l'amidon, le nitrure de bore, les micro sphères creuses polymériques expansées telles que celles de chlorure de polyvinylidène/acrylonitrile comme l'Expancel® (Nobel Industrie), les poudres acryliques telles que le polytrap® (Dow Corning), les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (SILICA BEADS® de MAPRECOS), les microcapsules de verre ou de céramique, les savons métalliques dérivés d'acides organiques carboxyliques

ayant de 8 à 22 atomes de carbone, de préférence de 12 à 18 atomes de carbone, par exemple le stéarate de zinc, de magnésium ou de lithium, le laurate de zinc, le myristate de magnésium.
Les charges peuvent représenter de 0,1 à 25 %, et mieux de 1 à 20 % en poids du poids total de la composition.

La composition de l'invention peut comprendre, en outre, tout additif usuellement utilisé en cosmétique tels que les antioxydants, les conservateurs, les parfums, les neutralisants, les gélifiants hydrophiles, les épaississants, les vitamines, les fibres et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Les gélifiants hydrophiles utilisables dans les compositions selon l'invention peuvent être choisi parmi :

les homo- ou copolymères d'acides acrylique ou méthacrylique ou leurs sels et leurs esters et en particulier les produits vendus sous les dénominations VERSICOL F® ou VERSICOL K® par la société ALLIED COLLOID, ULTRAHOLD 8® par la société CIBA-GEIGY, les acides polyacryliques de type SYNTHALEN K,
les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,
les copolymères acide polyacryliques/acrylates d'alkyle de type PEMULEN,
l'AMPS (Acide polyacrylamidométhyl propane sulfonique neutralisé partiellement à l'ammoniaque et hautement réticulé) commercialisé par la société CLARIANT,
les copolymères AMPS/acrylamide de type SEPIGEL® ou SIMULGEL® commercialisés par la société SEPPIC, et
les copolymères AMPS/méthacrylates d'alkyle polyoxyéthylénés (réticulés ou non), et leurs mélanges.

Les polymères filmogènes hydrosolubles cités plus haut peuvent également jouer le rôle de gélifiant hydrosoluble.
Le polymère gélifiant hydrosoluble peut être présent dans la composition selon l'invention en une teneur en matières sèches allant de 0,01 % à 60 % en poids, de préférence de 0,5 % à 40 % en poids, mieux de 1 % à 30 % en poids, voire de 5 à 20 % en poids par rapport au poids total de la composition.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.
En particulier, les fibres ont une longueur allant de 1 μm à 10 mm, de préférence de 0,1 mm à 5 mm et mieux de 0,3 mm à 3mm .
Les fibres utilisables dans la composition de l'invention peuvent être choisies parmi les fibres rigides ou non rigides, elles peuvent être d'origine synthétique ou naturelle, minérale ou organique.
A titre de fibres utilisables dans la composition selon l'invention, on peut citer les fibres non rigides telles que les fibres de polyamide (Nylon® ) ou les fibres rigides telles que les fibres de polyimide-amide comme celles vendues sous les dénomination "KERMEL", " KERMEL TECH" par la société RHODIA ou de poly-(p-phénylène-téréphtalamide) (ou d'aramide) notamment vendues sous la dénomination Kevlar ® par la société DUPONT DE NEMOURS.

De préférence, la composition selon l'invention est un mascara.

Les compositions selon l'invention peuvent être préparées selon des méthodes connues de l'homme du métier.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel

essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment sont incorporés par référence dans la présente demande.

Les exemples qui suivent sont présentés à titre illustratif et non limitatif de l'invention.
Les quantités indiquées sont en pourcentage pondéral et exprimées par rapport au poids total de la composition, sauf instructions contraires.

**Exemples 1 et 2: Mascaras**

On a préparé un mascara selon l'invention comprenant une cire de paraffine (exemple 2) et un mascara selon l'art antérieur (exemple 1) comprenant une cire polaire (cire de candellila).

| | Exemple 1 (hors invention) | Exemple 2 |
|---|---|---|
| Hydroxyéthyl cellulose | 0,9 | 0,9 |
| Triéthanolamine | 2,4 | 2,4 |
| Acide stéarique | 5,8 | 5,8 |
| Cire de candellila | 30 | |
| Cire de paraffine (CERAFINE 56/58 de BAERLOCHER) | | 30 |
| Eau | Qsp100 | Qsp100 |

A teneurs égales, la cire aprotique permet l'obtention d'une composition qui présente une consistance moins élevée (texture liquide) que la composition comprenant une cire polaire (texture crémeuse plus consistante).

## Revendications

1. Composition de revêtement des fibres kératiniques comprenant un milieu aqueux continu cosmétiquement acceptable **caractérisée en ce qu'**elle comprend au moins une cire aprotique en une teneur supérieure à 25% en poids par rapport au poids total de la composition, ladite composition ayant un extrait sec supérieur ou égal à 40%.

2. Composition de revêtement des fibres kératiniques comprenant un milieu aqueux continu cosmétiquement acceptable **caractérisée en ce qu'**elle comprend au moins 20% d'au moins une cire aprotique et au moins un polymère filmogène choisi parmi les dérivés cellulosiques.

3. Composition de revêtement des fibres kératiniques comprenant un milieu aqueux continu cosmétiquement acceptable **caractérisée en ce qu'**elle comprend moins de 5% de cire protique.

4. Composition de revêtement des fibres kératiniques comprenant un milieu aqueux continu cosmétiquement acceptable **caractérisée en ce qu'**elle est exempte de cire protique.

5. Composition selon l'une des revendications précédentes, **caractérisée en ce que** la cire aprotique est une cire apolaire.

6. Composition selon l'une quelconque des revendications précédentes, **caractérisée en ce que** la cire aprotique est choisie parmi : les cires de paraffines, les cires microcristallines, l'ozokérite, la cérésine, les cires synthétiques comme les cires de polyméthylène, de polyéthylène, de propylène et leurs copolymères éthylène/propylène, les cire de polyoléfine issues de la polymérisation d'alpha-oléfines de formule générale R-CH=$CH_2$ dans laquelle R désigne un radical alkyle ayant de 10 à 50 atomes de carbone, les cires de Fischer-Tropsch et leurs mélanges.

7. Composition selon l'une quelconque des revendications 2 à 6, **caractérisée en ce que** la cire aprotique représente de 20 à 60 % en poids par rapport au poids total de la composition.

8. Composition selon l'une quelconque des revendications 1 à 6, **caractérisée en ce que** la cire aprotique représente

de 25 à 60 % en poids, notamment de 27 à 50 % en poids, et en particulier de 28 à 45 % en poids par rapport au poids total de la composition.

**9.** Composition selon l'une quelconque des revendications précédentes, **caractérisée en ce que** milieu aqueux est présent en une teneur allant de 0,1 % à 95 % en poids, par rapport au poids total de la composition, de préférence allant de 1 % à 80 % en poids.

**10.** Composition selon l'une quelconque des revendications précédentes, **caractérisée en ce qu'**elle comprend au moins un agent tensioactif.

**11.** Composition selon la revendication 10, **caractérisée en ce que** ledit tensioactif est un tensioactif anionique choisi parmi les sels d'acides gras en $C_{16}$-$C_{30}$ ; les sels d'acides gras polyoxyéthoxylénés ; les esters phosphoriques et leurs sels ; les alkyléthersulfates ; les sulfosuccinates ; les iséthionates et les acylglutamates et leurs mélanges.

**12.** Composition selon la revendication 10 ou 11, **caractérisée en ce que** le tensioactif ionique comprend au moins le stéarate de triéthanolamine et/ou le stéarate d'amino-2 méthyl-2 propane di-ol-1,3.

**13.** Composition selon l'une quelconque des revendications 10 à 12, **caractérisée en ce que** le tensioactif représente de 0,1 à 30 % en poids par rapport au poids total de la composition, mieux de 1 à 15 % et mieux de 2 à 10 %.

**14.** Composition l'une quelconque des revendications précédentes, **caractérisée en ce qu'**elle comprend un polymère filmogène choisi parmi les polymères synthétiques, de type radicalaire ou de type polycondensat, les polymères d'origine naturelle et leurs mélanges.

**15.** Composition la revendication 14 **caractérisée en ce que** le polymère filmogène est choisi parmi les polymère de cellulose tels que les alkylcelluloses, les hydroxyalkylcelluloses les carboxyalkylcelluloses et leurs mélanges.

**16.** Composition selon la revendication 14 ou 15, **caractérisée en ce que** le polymère filmogène est présent en une teneur en matières sèches allant de 0,1 % à 60 % en poids par rapport au poids total de la composition, de préférence de 0,5 % à 40 % en poids, et mieux de 1 % à 30 % en poids.

**17.** Composition selon l'une des revendications précédentes, **caractérisée en ce qu'**elle comprend une matière colorante.

**18.** Composition selon la revendication 17, **caractérisée en ce que** la matière colorante est présente en une teneur allant de 0,01 % à 30 % en poids, par rapport au poids total de la composition.

**19.** Composition selon l'une des revendications précédentes, **caractérisée en ce qu'**elle présente un extrait sec supérieur ou égal à 42% en poids, mieux, supérieur ou égal à 45% et encore mieux supérieur ou égal à 47% par rapport au poids total de la composition, pouvant aller jusqu'à 70% en poids.

**20.** Composition selon l'une des revendications 1 à 19, **caractérisée en ce qu'**elle est un mascara.

**21.** Utilisation d'une composition selon l'une des revendications 1 à 20, pour obtenir un maquillage chargeant des fibres kératiniques et notamment des cils.

**22.** Utilisation d'au moins une cire aprotique dans une composition comprenant un milieu aqueux continu cosmétiquement acceptable, pour obtenir un maquillage chargeant des fibres kératiniques et notamment des cils et des sourcils et un dépôt lisse et homogène sur lesdites fibres.

**23.** Procédé cosmétique de soin ou de maquillage des fibres kératiniques comprenant l'application sur les dites fibres kératiniques d'une composition selon l'une quelconque des revendications 1 à 20.